(19) Europäisches Patentamt
European Patent Office
Office européen des brevets

(11) EP 3 301 084 B1

(12) **EUROPÄISCHE PATENTSCHRIFT**

(45) Veröffentlichungstag und Bekanntmachung des Hinweises auf die Patenterteilung:
**12.12.2018 Patentblatt 2018/50**

(51) Int Cl.:
***C07C 45/75*** *(2006.01)* ***C07C 47/21*** *(2006.01)*

(21) Anmeldenummer: **16191892.5**

(22) Anmeldetag: **30.09.2016**

# (54) KONTINUIERLICHES HERSTELLUNGSVERFAHREN FÜR 2-METHYLENALKANALE

CONTINUOUS PROCESS FOR PRODUCING 2-METHYLENE ALDEHYDES

PROCÉDÉ DE FABRICATION CONTINU DES ALDÉHYDES 2-MÉTHYLÈNÉS

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**04.04.2018 Patentblatt 2018/14**

(73) Patentinhaber: **OXEA GmbH**
**46147 Oberhausen (DE)**

(72) Erfinder:
- **Schalapski, Kurt**
  **46147 Oberhausen (DE)**
- **Rahe, Jan-Henry**
  **45701 Herten Westerholt (DE)**
- **Balzarek, Dr. Christoph**
  **47803 Krefeld (DE)**
- **Meier, Dr. Gregor**
  **47057 Duisburg (DE)**
- **Strutz, Dr. Heinz**
  **47445 Moers (DE)**

(74) Vertreter: **Michalski Hüttermann & Partner Patentanwälte mbB**
**Speditionstraße 21**
**40221 Düsseldorf (DE)**

(56) Entgegenhaltungen:
**EP-A1- 2 883 859**
**EP-A1- 2 998 284**
**WO-A1-2014/170223**
**WO-A2-2013/079614**
**CN-A- 105 693 491**
**DE-A1- 3 744 212**
**DE-A1- 19 957 522**
**US-A- 4 263 460**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die vorliegende Erfindung betrifft die kontinuierliche Herstellung von 2-Methylenalkanalen in einem Rohrreaktor, wobei in 2-Position nicht verzweigte Alkanale unter laminaren Strömungsbedingungen mit Formaldehyd unter Säure-Base-Katalyse in Gegenwart von sekundären Aminen und Carbonsäuren umgesetzt werden.

2-Methylenalkanale sind auf Grund ihrer hohen Funktionalität wertvolle Zwischenprodukte in der industriellen organischen Chemie. Durch Selektivhydrierung der Doppelbindung können 2-Methylalkanale erhalten werden, welche in der Riechstoffindustrie eine bedeutende Rolle spielen. Eine weitere wichtige Umsetzung dieser Substanzklasse ergibt sich beispielsweise über die Oxidation der Aldehydfunktion zu dann ungesättigten Carbonsäuren, welche im großen Maßstab für die Herstellung von Kunststoffen, Schmierölen oder Textilhilfsmitteln Verwendung finden.

Es ist bekannt, 2-Methylenalkanale durch Umsetzung unverzweigter n-Aldehyde mit Formaldehyd herzustellen. Die Umsetzung erfolgt in Gegenwart sekundärer Amine in Form einer Mannich-Kondensationsreaktion, wobei sich unter Wasserabspaltung zunächst eine Mannichbase bildet

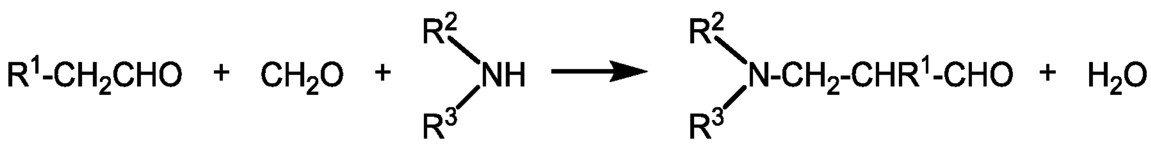

,

aus der man unter Abspaltung des sekundären Amins letztendlich das 2-Methylenalkanal erhält:

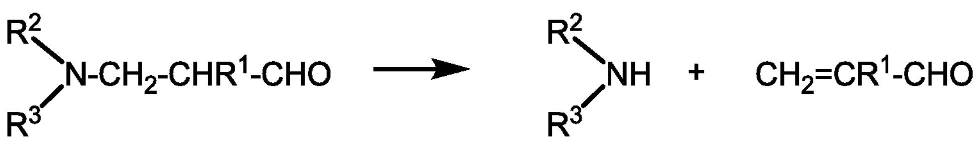

.

(Methoden der Organischen Chemie, Houben Weyl, Georg Thieme Verlag, 4. Auflage 1954, Band VII, Teil 1, Seiten 93-94).

Die Kondensationsreaktion kann dabei als solche wahlweise nur mit den in oben angegebenen Reaktionsschemata aufgeführten Substanzklassen erfolgen oder aber durch Zugabe von Säuren oder weiteren Basen katalysiert werden.

Die DE 2855 506 A1 offenbart eine Möglichkeit zur Umsetzung von Alkanalen nur in Gegenwart katalytischer Mengen sekundärer Amine.

Die WO199320034 A1 beschreibt die Aldolisierung von C3- bis C10-Aldehyden in einem kontinuierlich betriebenen Rührkessel mit anschließender Destillation. Es werden substituierte Acroleine hergestellt, wobei als zusätzliche Katalysatoren Hydroxide oder Carbonate verwendet werden.

Eine säure-base-katalysierte Herstellungsweise ist beispielsweise in der DE3744212 A1 offenbart. In dem dort beschriebenen Verfahren wird ein C5-Alkanalgemisch, das bei der Hydroformulierung von isomeren Butenen anfällt, innerhalb einer Reaktivdestillation mit einer wässrigen Formaldehydlösung in Gegenwart eines sekundären Amins und einer Mono-, Di- oder Polycarbonsäure umgesetzt.

Ebenso vielfältig wie die Wahl der möglichen Reaktionsbedingungen sind auch die zur Herstellung einsetzbaren Fahrweisen und Reaktortypen. So finden sich in der Patentliteratur sowohl batch- als auch kontinuierliche Prozesse, wobei die Reaktion unter anderem in Rührkesseln, Rührkesselkaskaden und auch Rohrreaktoren durchgeführt wird. Ein Beispiel für eine wahlweise kontinuierliche oder Batch-Umsetzung in einem Rührkessel ist in der oben genannten DE 2855 506 A1 angeführt. Eine kontinuierliche Umsetzung in einem Rohrreaktor ist beispielsweise in der DE 199 57 522 A1 beschrieben.

Weitere Verfahren zur Herstellung von Methylenalkanalen über Mannich-Reaktionen unter den unterschiedlichsten Prozessbedingungen werden beispielsweise in der EP 2 998 284 A1, WO 2014/170223 A1, EP 2 883 859 A1, DE 199 57 522, US 4 263 460 und der WO 2013/079614 A1 beschrieben. Zudem zeigt die CN 105 693 491 die Durchführung von Mannichreaktionen unter isothermen Bedingungen in einem Mikrokanalysystem.

Für die Umsetzung der eingesetzten Aldehyde mit Formaldehyd in Gegenwart eines Katalysators werden typischerweise Rührkessel verwendet, um eine intensive Durchmischung der organischen und der wässrigen Phase sicherzustellen. Die Reaktion in Rührkesseln ist jedoch nachteilig, da die diskontinuierliche Reaktionsführung neben Aufheiz- und Abkühlvorgänge auch einen ungleichmäßigen Anfall an Reaktionswärme zeigt, was zu suboptimalen Reaktionsergebnissen führen kann. Zudem ist der Betrieb mechanisch bewegter Teile typischerweise wartungsintensiv

und reparaturanfällig. Andererseits erscheint eine kontinuierliche Reaktionsführung in einem Rührkessel oder einer Rührkesselkaskade auf Grund des flüssigen Mehrphasensystems als problematisch, da ein unerwünschter einseitiger Austrag einer Phase zur Anreicherung der anderen Phase führen kann und damit Nachteile durch Schwankungen der mittleren Verweilzeit in Bezug auf Umsatz, Selektivität und Raum-Zeit-Ausbeute mit sich bringt.

Die Aufgabe der vorliegenden Erfindung besteht darin, ein kontinuierliches Herstellungsverfahren für 2-Methylenalkanale bereitzustellen, welches einen nur geringen technischen Aufwand erfordert, wenig energieintensiv ist und sich durch hohe Raum-Zeit-Ausbeuten auszeichnet.

Gelöst wird diese Aufgabe durch ein Verfahren nach Anspruch 1. Bevorzugte Ausgestaltungen des Verfahrens werden in den Unteransprüchen wiedergegeben.

Erfindungsgemäß gelöst wird die Aufgabe durch ein Verfahren zur Herstellung von 2-Methylenalkanalen der allgemeinen Formel I

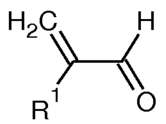

Formel I,

worin $R^1$ für einen aliphatischen Rest steht, durch Umsetzung von Alkanalen nach der allgemeinen Formel II

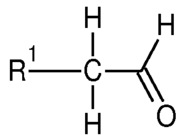

Formel II,

mit einer wässrigen Formaldehydlösung in Gegenwart mindestens eines sekundären Amins und mindestens einer Carbonsäure, wobei die Edukte in einem flüssig/flüssig Zweiphasensystem zur Reaktion gebracht werden, und wobei die Reaktion kontinuierlich innerhalb eines Rohrreaktors unter laminaren Strömungsbedingungen mit einer Reynolds-Zahl von größer oder gleich 10 und kleiner oder gleich 2320 durchgeführt wird. Überraschenderweise wurde gefunden, dass innerhalb eines kontinuierlichen Verfahrens in einem Rohrreaktor in strikt laminarer Fahrweise unter Säure-Base-Katalyse sehr hohe Raum-Zeit-Ausbeuten an gewünschten 2-Methylenalkanen realisierbar sind. Dadurch können die Produktionsanlagen kleiner dimensioniert werden, und es lassen sich unter geringen Investitions- und Betriebskosten vorteilhafterweise hohe Produktmengen erhalten. Dieses Ergebnis ist deshalb überraschend, da im Stand der Technik technische Lösungen als besonders bevorzugt herausgestellt werden, in denen sich große Phasengrenzflächen zwischen wässriger und organischer Phase einstellen. Nach den allgemeinen Regeln der Reaktionskinetik sollen große Phasengrenzflächen zu einem hohen Stoffaustausch zwischen organischer und wässriger Phase beitragen und konsequenterweise so zu hohen Ausbeuten führen. Zum Erhalt dieser großen Phasengrenzflächen werden turbulente Fahrweisen vorgeschlagen, welche eine konstant gute Durchmischung der organischen und der wässrigen Phase gewährleisten sollen. Diese gute Durchmischung ist im Falle von Rührkesseln und/oder -Kaskaden, wie schon oben erwähnt, mit einem hohen technischen Aufwand und hohen Betriebskosten verbunden. Eine äquivalente Betrachtung sollte auch für Rohrreaktoren gelten, wobei auch hier ein turbulentes Strömungsregime zu einer höheren Phasengrenzfläche und somit zu höheren Umsätzen beitragen sollte. Darüber hinaus ermöglicht typischerweise ein turbulentes Regime eine deutlich bessere Wärmeabfuhr, was zur Selektivitätssteuerung der betrachteten exothermen Reaktion durchaus hilfreich ist. Ohne durch die Theorie gebunden zu sein, wird der Erhalt einer hohen Raum-Zeit-Ausbeute unter "nur" laminaren Strömungsbedingungen, also bei einer im Vergleich zu turbulenten Fahrweise potentiell geringeren Phasengrenz-/Austauschfläche, insbesondere durch die erfindungsgemäß eingesetzte Säure-Base-Katalyse erreicht. Anscheinend sind die erfindungsgemäß einsetzbaren Carbonsäuren in der Lage, in dem organisch/wässrigen Zweiphasensystem Grenzflächen zu stabilisieren und zu einem hohen Stoffaustausch beizutragen. Dies könnte partiell auch durch den amphiphilen Charakter der Carbonsäuren verursacht sein, welcher zu einer Stabilisierung in einem laminaren Strömungsregime gebildeter Phasengrenzflächen beitragen könnte. Darüber hinaus erlaubt das gewählte Mehrphasenströmungsregime eine ausreichende Stabilität der Reaktionstemperatur, was im Hinblick auf die strikt laminare Fahrweise nicht selbstverständlich ist.

Die einsetzbaren Alkanale nach der Formel II können generell Aldehyde mit einer C3-C15 Kohlenstoffzahl ($R^1$ ist ein aliphatischer Organylrest mit 1 bis 13 C-Atomen) sein. Die Ausgangsverbindungen können daher als kurze oder mittlere Aldehyde aufgefasst werden. Insbesondere können Aldehyde verwendet werden, welche aus bekannten Hydroformylierungsreaktion oder Oxo-Reaktion der entsprechenden, um ein Kohlenstoffatom verminderten Olefine erhalten werden. Bei dieser Hydroformylierungsreaktion wird das Einsatzolefin mit einem Gemisch aus Kohlenmonoxid und

Wasserstoff in Gegenwart von Übergangsmetallkatalysatoren, wie Kobalt- oder Rhodiumkatalysatoren, umgesetzt. Die Reaktion kann entweder ohne oder mit Organophosphorverbindungen, beispielsweise Triphenylphosphin, als Komplexliganden durchgeführt werden und ist in der technischen Literatur ausführlich beschrieben (Ullmans's Encyclopedia of Industrial Chemistry, 5th Ed., 1985, VCH Verlagsgesellschaft mbH, Vol. A1, Seiten 326-331; 5th Ed., 1991 VCH Verlagsgesellschaft mbH, Vol. A18, Seiten 321-327). Bei der Hydroformylierungsreaktion von endständigen Olefinen fällt ein Isomerengemisch aus Alkanalen an, wobei neben den Alkanalen der allgemeinen Formel II auch 2-Methylalkanale sowie weitere Alkanale entstehen. Die relativen Anteile dieser Isomeren hängen von den Hydroformylierungsbedingungen ab.

Bei dem in dem erfindungsgemäßen Verfahren einzusetzenden Alkanalen mit 3 bis 15 Kohlenstoffatomen ($R^1$ ist ein aliphatischer Organylrest mit 1 bis 13 C-Atomen) handelt es sich üblicherweise um die Produkte aus der Hydroformylierung von Olefinen, beispielsweise um die Hydroformylierungsprodukte aus der Umsetzung von Hexen-1, Hepten-1, Octen-1, Nonen-1, Decen-1, Undecen-1 oder Dodecen-1. Es lassen sich nach dem erfindungsgemäßen Verfahren relativ kurzkettige Aldehyde mit 3 bis 5 Kohlenstoffatomen im Molekül, wie beispielsweise Propionaldehyd, n-Butyraldehyd, n-Pentanal oder 3-Methylbutanal umsetzen. Weiterhin können auch Aldehyde mit höheren C-Zahlen wie beispielsweise n-Heptanal, n-Octanal, n-Nonanal, 3,5,5,-Trimethylhexanal-1, n-Decanal, n-Undecanal, n-Dodecanal oder n-Tridecanal umgesetzt werden. Die einzusetzenden Alkanale müssen aber mindestens ein Alkanal mit zwei Wasserstoffatomen an dem zur Carbonylfunktion benachbarten C-Kohlenstoffatom (2-Position, $\alpha$-Stellung) enthalten, damit es zur Bildung der 2-Methylenalkanale kommen kann.

Weiterhin wurde auch überraschenderweise gefunden, dass sich über obiges Verfahren auch Alkanal-Mischungen umsetzen lassen, welche mehrere Edukt-Alkanale nach der Formel II oder Mischungen unterschiedlicher Alkanale umfassen. So ist das beschriebene Verfahren zum Beispiel anwendbar auf ein Gemisch von n-Undecanal und 2-Methyldecanal, bei dem nur der n-Aldehyd abreagiert, der Methyl-verzweigte Aldehyd sich weitestgehend inert verhält. Insbesondere kann das Verfahren auch dazu Verwendung finden, selektiv den Siedepunkt von Alkanalen mit zwei Wasserstoffatomen in alpha-Position relativ zur Carbonylgruppe durch Überführung in die entsprechenden 2-Methylenalkanale zu erhöhen, wobei diese aufgrund ihres dann höheren Siedepunktes sich leichter aus Gemischen abtrennen lassen. Dies kann zum Beispiel bei der Auftrennung eines 2-Methylbutanal und 3-Methylbutanal-Gemischs von Bedeutung sein. Besonders gute Ergebnisse mit hohen Raum-Zeit-Ausbeuten ergeben sich aber in Anwesenheit von nur einem Edukt nach Formel II in der Reaktionslösung.

In der Formel I und II steht $R^1$ für einen aliphatischen Rest. Gemeint sind damit lineare, verzweigte oder zyklische gesättigte Kohlenwasserstoffreste mit einer Kohlenstoffanzahl von C1-C13. Substitutionen in der Kette zum Beispiel mit einer oder mehreren Halogenfunktion(en) oder einer oder mehreren Hydroxylgruppe(n) sind möglich.

Die Umsetzung von Alkanalen nach der allgemeinen Formel II beinhaltet das zur Reaktion bringen des Edukt-Alkanals unter Säure-Base-Katalyse unter Gegenwart eines sekundären Amins und einer Carbonsäure zu dem entsprechenden 2-Methylenalkanal.

Als wässrige Formaldehydlösung lassen sich Formalinlösungen beispielsweise in einer Konzentration von 20 - 49 Gew.-%, vorzugsweise von 25 bis 35 Gew.-% einsetzen. Bezogen auf ein Mol Edukt Alkanal der Fomel II kann dabei Formaldehyd beispielsweise in stöchiometrischer Menge verwendet werden. Die Reaktion kann aber auch in einem Formaldehyd-Überschuss, vorzugsweise bis zu 20 Mol-% und insbesondere bis zu 10 Mol-%, gefahren werden.

Das zur Reaktion bringen erfolgt dabei in Gegenwart mindestens eines sekundären Amins. Als sekundäre Amine kommen zweckmäßig solche der Formel

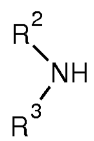

in Betracht, worin $R^2$ und $R^3$ gleich oder verschieden sein können und einen Alkylrest mit 1 bis 12, vorteilhaft 1 bis 10, bevorzugt 1 bis 6 Kohlenstoffatome bedeuten, der gegebenenfalls mit einem oder mehreren Heteroatomen, bevorzugt mit Hydroxyl und/oder sekundärem bzw. tertiärem Amin substituiert sein kann. $R^2$ und $R^3$ können ebenfalls mit dem benachbarten Kohlenstoffatom auch Glieder eines vorteilhaft 5- oder 6-gliedrigen Ringes, der noch ein Stickstoff- oder Sauerstoffatom enthalten kann, bilden. So kommen als sekundäre Amine im Einzelnen in Betracht: N-Methyl-, N-Ethyl-, N-Propyl-, N-Isopropyl-, N-Butyl-, N-sek.-Butyl, N-tert.-Butyl-, N-Pentyl-, N-Hexyl-, N-Heptyl-, N-Octyl-, N-Nonyl-, N-Decyl-(2-hydroxyethylamin); entsprechende durch einen vorgenannten Substituenten gleich oder unterschiedlich zweimal substituierte Amine; Piperidin, Morpholin, Pyrrolidin, Piperazin, N-Methylpiperazin; N,N'-Dimethylethylendiamin.

Bevorzugt können (2-Hydroxyethyl)-N-methylamin, N-Ethyl-(2-hydroxyethyl)amin, (2-Hydroxyethyl)-N-isopropylamin, N-Butyl-(2-hydroxyethyl)amin, (2-Hydroxypropyl)-N-isopropylamin, (3-Hydroxypropyl)-N-isopropylamin, N-Butyl-(2-hydroxypropyl)amin, N-Butyl-(3-hydroxypropyl)amin, (2-Hydroxypropyl)-N-isobutylamin, (3-Hydroxypropyl)-N-isobutylamin, N,N-Bis(2-hydroxyethyl)amin, Bis(2-hydroxypropyl)amin, Bis(3-hydroxypropyl)amin, N,N'-Bis(2-Hydroxyethyl)ethylendiamin, Piperazin, N-Methylpiperazin, sowie die Dialkylamine Di-n-propylamin, Di-n-pentylamin, Di-n-hexylamin und insbesondere bevorzugt Di-n-butylamin eingesetzt werden.

Die Reaktion erfolgt dabei säure-base-katalysiert unter Anwesenheit mindestens einer Carbonsäure. Als Carbonsäuren verwendet man üblicherweise aliphatische Mono-, Di- und Polycarbonsäuren mit 2 bis 10 Kohlenstoffatomen. Dicarbonsäuren und Polycarbonsäuren, darunter vorzugsweise Tricarbonsäuren, können auch aromatische und aliphatische Carbonsäuren sein. Vorzugsweise führt man die Umsetzung in Gegenwart von Monocarbonsäuren durch. Geeignete Monocarbonsäuren sind beispielsweise Essigsäure, Propionsäure, n-Buttersäure, iso-Buttersäure, 2-Ethylbuttersäure, 2-Methylpentansäure, 2-Ethylhexansäure und Isononansäure. Zu den Polycarbonsäuren, die in dem erfindungsgemäßen Verfahren eingesetzt werden können, zählen Pimelinsäure, Korksäure, Acelainsäure, Sebacinsäure, Nonandicarbonsäure, Decandicarbonsäure, Butantetracarbonsäure, Pentan-1,3,5-Tricarbonsäure, 3-Hydroxyglutarsäure, Zuckersäure, $\alpha,\alpha'$-Dihydroxy-adipinsäure, bevorzugt Oxalsäure, Bernsteinsäure, Glutarsäure, Adipinsäure, Apfelsäure, Weinsäure, Butan-1,2,4-tricarbonsäure, 3-Ethylpentan-1,3,5-tricarbonsäure, Zitronensäure, Trimellitsäure, Butantetracarbonsäure, Pyromellitsäure, Terephthalsäure, Isophthalsäure und Fumarsäure.

In einer besonders bevorzugten Ausführung kann die Umsetzung des Alkanals mit Formaldehyd in Gegenwart von Di-n-Butylamin und n-Buttersäure als Katalysator erfolgen.

Die Umsetzung der Edukte erfolgt in einem flüssig/flüssig Zweiphasensystem. Bedingt durch das Zusammengeben einer wässrigen und einer organischen Phase bildet sich ein zweiphasiges System aus, in welchem die organische Phase in der wässrigen Phase dispergiert vorliegt. Ohne durch die Theorie gebunden zu sein wird angenommen, dass aufgrund der Verteilungskoeffizienten die erfindungsgemäß einsetzbaren Carbonsäuren und das sekundäre Amin hauptsächlich in der organischen Phase gelöst vorliegen. Im Rahmen der Reaktion geht Formaldehyd dann in die organische Phase über und reagiert mit dem Alkanal. Entsprechend der Löslichkeit von Formaldehyd im Wasser weist die wässrige Phase einen Restgehalt von Formaldehyd auf.

Die Reaktion erfolgt erfindungsgemäß kontinuierlich innerhalb eines Rohrreaktors. Dies bedeutet, dass die Zuführung der Edukte und die Abführung der Produkte kontinuierlich erfolgen, beispielsweise als ununterbrochener Strom der unterschiedlichen Lösungen in und aus dem Rohrreaktor. Es kann sich eine konstante, laminare Fließgeschwindigkeit des Zweiphasensystems aufbauen. Das Zweiphasensystem kann beispielsweise dadurch ausgebildet werden, indem getrennte Eduktströme gleichzeitig in den Bodenbereich eines Rohrreaktors eingespeist werden. Im Bodenbereich können Einbauten vorgesehen werden, welche eine tropfenförmige Verteilung der eintretenden Reaktanden sicherstellen. Als Einbauten können beispielsweise Düsen, poröse Sinterplatten oder Lanzen installiert sein. Die Konstruktionsweise eines Rohrreaktors ist dem Fachmann prinzipiell bekannt.

Die Reaktion wird innerhalb eines Rohrreaktors unter laminaren Strömungsbedingungen mit einer Reynolds-Zahl von größer oder gleich 10 und kleiner oder gleich 2320 durchgeführt. Die Reynolds-Zahl ist eine dimensionslose Kennzahl und beschreibt die Charakteristik einer Strömung. Anhand der Reynolds-Zahl lässt sich erkennen, ob eine turbulente oder eine laminare Strömung (Pfropfenströmung) vorliegt. Die kritische Reynolds-Zahl charakterisiert dabei den Übergangsbereich von einer laminaren zu einer turbulenten Strömung. Zur Abschätzung der Reynolds-Zahl für das hier betrachtete flüssig/flüssig Zweiphasensystem wird die mittlere Strömungsgeschwindigkeit aller Reaktanden auf den Strömungsquerschnitt (homogenes Modell) betrachtet (Transportvorgänge in der Verfahrenstechnik: Grundlagen und apparative Umsetzungen, Matthias Kraume, Springer Berlin Heidelberg, Auflage 2, 2012; WO 2010/105892 A1).

Um den laminaren Strömungszustand innerhalb dieses Reynolds-Zahlenbereiches im Rohrreaktor sicherzustellen, sind der Massenstrom der Reaktanden sowie die Dichte und die dynamische Viskosität der Reaktionsmischung mit dem hydraulischen Innendurchmesser des Rohrreaktors in Beziehung zu setzen und zu kontrollieren. Dies erfolgt derart, dass der geforderte Reynolds-Zahlenbereich, welcher einen laminaren Strömungszustand anzeigt, nicht überschritten wird. Der formelmäßige Zusammenhang zur Berechnung der Reynolds-Zahl ergibt sich gemäß Gleichung (1)

$$Re = w\, \rho\, d / \eta \quad (1)$$

mit w = Massenstrom [kg/h] der Reaktanden, $\rho$ = Dichte [kg/m$^3$] der Reaktionsmischung, d = hydraulischer Innendurchmesser des Rohrreaktors [m] und $\eta$ = dynamische Viskosität [Pa·s] der Reaktionsmischung (VDI Wärmeatlas, 7. Auflage 1994, Lb1, Gl. (2); Grundlagen der Einphasen- und Mehrphasenströmungen, Heinz Bauer, Verlag Sauerländer, Aarau und Frankfurt am Main, 1971). Die Reynolds-Zahl für eine Rohrströmung, an welcher eine laminare in eine turbulente Strömung übergeht, wird mit 2320 angegeben. Unter der Annahme eines homogenen Modells kann auch hier diese Unterteilung gelten. Unterhalb dieser Zahl liegt ein laminarer Strömungszustand vor. Erfindungsgemäß liegt im Rohrreaktor eine laminare Strömung vor, wenn in mindestens 80 %, vorzugsweise in mindestens 90 % oder noch bevorzugter in 100 % des Reaktorvolumens ein laminares Strömungsprofil vorherrscht. Es wurde überraschenderweise gefunden, dass unter diesen Bedingungen exzellente Raum-Zeit-Ausbeuten möglich sind. Für den Fall eines 100%igen

laminaren Strömungsprofiles werden die Bereiche des Einspeisens der Edukt- und des Ausführens des Produktstromes außen vor gelassen, welche kurzzeitig und räumlich eng begrenzt eine turbulente Charakteristik zeigen könnten.

In einer bevorzugten Ausführungsform des Verfahrens kann die Reynolds-Zahl innerhalb des Rohrreaktors konstant gehalten werden. Es hat sich als besonders günstig erwiesen, wenn das Strömungsprofil der Reaktionsmischung über weite Bereiche rein laminar ist. Da Reaktoreinbauten, wie statische Mischer oder Kolonnenpackungen, die Turbulenz im Rohrreaktor erhöhen könnten, ist ihre Verwendung weniger zu empfehlen. Ihr Einsatz ist jedoch erfindungsgemäß nicht ausgeschlossen, solange der Massenstrom der Reaktanden unter laminaren Bedingungen durch den Rohrreaktor strömt. Hingegen können Kühlschlangen oder Kühlfinger in dem Rohrreaktor installiert sein, an denen der Massenstrom der Reaktanden ohne Störung des laminaren Zustands vorbeiströmt. Insbesondere vorteilhaft kann der Rohrreaktor ohne Einbauten betrieben werden. Dazu kann die wässrige Formaldehydlösung, das Alkanal und die organische Lösung des Katalysators in einem dem Rohreaktor vorgeschaltetem Mischelement zu einem Gesamtmassenstrom vermischt werden, der dann anschließend auf den Boden eines Rohrreaktors geführt wird. Beispielsweise können dazu kommerziell erhältliche statische Mischelemente verwendet werden (beispielsweise Sulzer- oder Kenicks-Mischer).

In einer weiteren Ausgestaltung des Verfahrens kann das flüssig/flüssig Zweiphasensystem durch getrenntes Einspeisen im Gleichstrom mindestens einer organischen und mindestens einer wässrigen Phase in den Rohrreaktor ausgebildet werden. Zur schnellen und möglichst definierten Ausbildung eines laminaren Strömungsprofils können die organische und das Formaldehyd in wässriger Phase getrennt und im Gleichstrom in den Reaktor gegeben werden. Auf diese Art und Weise kann eine gerichtete laminare Strömung ausgebildet werden, in welcher eine ausreichend hohe Phasengrenzfläche vorhanden ist, die eine hohe Raum-Zeit-Ausbeute bei gleichzeitig hoher Selektivität ermöglicht.

Nach einer bevorzugten Ausführungsform des Verfahrens kann die Carbonsäure zusammen mit dem sekundären Amin in einem organischen Lösungsmittel gelöst in den Reaktor eingespeist werden. Diese Ausgestaltung mit vorgelöstem Katalysator bestehend aus Carbonsäure und Amin kann dazu beitragen, dass innerhalb der Umsetzung im Rohrreaktor höhere Raum-Zeit-Ausbeuten erhalten werden. Ohne durch die Theorie gebunden zu sein, kann das Vorlösen der Säure und des Amins zu einer schnelleren Gleichgewichtsverteilung dieser Komponenten in der organischen Phase und auch zu einer schnelleren Einstellung des gesamten Phasengleichgewichts führen. Das System wird dadurch stabilisiert und innerhalb einer kürzeren Reaktionszeit werden höhere Umsätze zugänglich. Als organische Lösungsmittel eignen sich beispielsweise Alkohole, Ester oder Ether, insbesondere mit 6 bis 12 Kohlenstoffatomen. Diese Lösungsmittel führen zu einer schnellen Verteilung der Säurekomponente und des Amins im Alkanal und diese Gruppe an Lösemitteln lässt sich nach der Reaktion relativ einfach, ohne großen Aufwand vom Hauptproduktstrom wieder abtrennen.

In einer weiteren Ausgestaltung kann das Lösungsmittel aus der Gruppe der Monoalkohole mit 6 bis 12 Kohlenstoffatomen oder Mischungen daraus ausgesucht sein. Zur Umsetzung gerade der kurz- bis mittelkettigen Aldehyde hat sich diese Gruppe an Vorlösemitteln als besonders geeignet erwiesen. Besonders geeignet sind Monoalkohole mit 6 bis 12 Kohlenstoffatomen wie Heptanol, n-Octanol, 2-Ethylhexanol, n-Nonanol oder n-Decanol. Besonders bewährt hat sich 2-Ethylhexanol. Die Konzentration an dem sekundären Amin in dem organischen Lösungsmittel kann im Allgemeinen von 30 bis 50 Gew.-% und an der Carbonsäure von 20 bis 40 Gew.-% betragen. Nach dem Eintritt der vergleichsweise konzentrierten Lösung in den Rohrreaktor vereinen sich die organische Lösung des Katalysators und das anwesende Alkanal zu der organischen Phase und es werden die gewünschten molaren Verhältnisse bezogen auf das Alkanal der Fomel II eingestellt.

Innerhalb eines zusätzlichen erfindungsgemäßen Aspekts des Verfahrens kann die Reaktion bei einer Temperatur von größer oder gleich 70 °C und kleiner oder gleich 150°C durchgeführt werden. Innerhalb dieses Temperaturbereiches lassen sich mit guten Selektivitäten hohe Raum-Zeit-Ausbeuten erhalten. Höhere Temperaturen können aus Betriebskostensicht und bezüglich der Bildung höhermolekularer Nebenprodukte nachteilhaft sein. Tiefere Temperaturen können zu einer unvorteilhaften Kinetik betragen, welche ungünstige Auswirkungen auf das Volumen des Rohrreaktors haben kann. Weiterhin bevorzugt kann die Reaktion bei einer Temperatur von größer oder gleich 90 °C und kleiner oder gleich 130 °C durchgeführt werden. Ggf. ist eine Verfahrensdurchführung unter Druck vorteilhaft, um Komponenten mit Siedepunkten unterhalb der gewünschten Reaktionstemperatur Rechnung zu tragen und Siedeverzüge zu vermeiden. Vorteilhafterweise kann die Reaktion mit einem Überdruck von 0,05; bevorzugt 0,10, weiterhin bevorzugt 0,15 und 0,20 MPa bis zu weniger als 1 MPa, bevorzugt weniger als 0,50 MPa betrieben werden. Diese Druckbereiche können effektiv Siedeverzüge vermeiden, eine gleichmäßige Fahrweise erlauben und dadurch zu einer weiteren Erhöhung der Ausbeuten beitragen.

Eine weitere Ausführungsform des Verfahrens kann sich dadurch ergeben, dass in dem Zweiphasensystem keine zusätzlichen oberflächenaktiven Substanzen vorliegen. Ohne durch die Theorie gebunden zu sein, kann insbesondere die erfindungsgemäße Säure-Base-Katalyse zu einer ausreichenden Stabilisierung des Zweiphasensystems beitragen, sodass zur Ausbildung einer über das gesamte Reaktorvolumens hinreichend großen Phasengrenzfläche selbst in einem laminaren Strömungsregime keinerlei weitere Zusatzstoffe nötig sind. Auf diese Art kann auf den Einsatz oberflächen-/grenzflächenaktiver Substanzen wie Tenside, Netzmittel oder ähnliches verzichtet werden. Dies kann die

Aufreinigung der Produkte erleichtern und vermindert die Gefahr einer Akkumulation dieser Substanzen im System.

In einer bevorzugen Ausgestaltung kann der Rohrreaktor mit einer Reynolds-Zahl von größer oder gleich 50 und kleiner oder gleich 500 betrieben werden. Wie durch die Beispiele gezeigt, können insbesondere im unteren Reynolds-Zahlenbereich mit einer laminaren Fahrweise sehr hohe Raum-Zeit-Ausbeuten erhalten werden. Dieser untere Bereich erfordert zudem nur einen geringen Energieeintrag zur Aufrechterhaltung und kann so zu geringeren Anlagen-Investitionskosten und Betriebskosten beitragen. Weiterhin bevorzugt kann der Rohrreaktor mit einer Reynolds-Zahl von größer oder gleich 100 und kleiner oder gleich 400 betrieben werden.

Innerhalb eines weiteren Verfahrensaspektes kann die Reaktorbelastung V/Vh (Volumen der Reaktionsmasse pro Reaktorvolumen und Zeit) größer oder gleich 3,0 $h^{-1}$ und kleiner oder gleich 42,0 $h^{-1}$ betragen. Überraschenderweise kann unter den angegebenen Reaktionsbedingungen trotz einer vergleichsweise hohen Reaktorbelastung, entsprechend einer geringeren Verweilzeit, bei gleichbleibend hoher Selektivität ein hoher Umsatz an Alkanal der Formel II erzielt werden. Die erfindungsgemäße Durchführung in einem Rohrreaktor unter laminaren Bedingungen erlaubt somit sehr hohe Raum-Zeit-Ausbeuten an dem gewünschten 2-Methylenalkanal. Außerhalb des angegebenen Bereiches könnte es gegebenenfalls zu einer nur ungenügenden Umsetzung oder zu einer vermehrten Hochsiederbildung kommen. Weiterhin bevorzugt kann das V/Vh größer oder gleich 5,0 $h^{-1}$ und kleiner oder gleich 30,0 $h^{-1}$ betragen.

Nach einer weiteren, bevorzugten Ausgestaltung des Verfahrens kann die Carbonsäure aus der Gruppe der aliphatischen oder aromatischen C2-C12 Monocarbonsäuren ausgesucht sein. Die Gruppe an kurz- bis mittelkettigen Monocarbonsäuren hat sich zur Stabilisierung des laminaren Systems und zum Erhalt hoher Raum-Zeit-Ausbeuten als besonders geeignet erwiesen. Ohne durch die Theorie gebunden zu sein, scheinen sich die Carbonsäuren sehr gut in die dispergierte Phase kurz- bis mittelkettiger Alkanale zu integrieren und in dieser diffundieren zu können. Dies kann auch zum Erhalt höherer Produktausbeuten beitragen.

In einer besonders bevorzugten Ausführung kann die Umsetzung des Alkanals mit Formaldehyd in Gegenwart von Di-n-Butylamin und Buttersäure als Katalysator erfolgen.

Innerhalb einer bevorzugten Ausgestaltung des Verfahrens kann das molare Verhältnis aus Alkanal, Formaldehyd im Formalin und sekundärem Amin im Bereich von 1 zu 1 zu 0,01 bis 1 zu 1,2 zu 0,07 liegen. Dieser Bereich molarer Verhältnisse zwischen Alkanal, Formaldehyd in der wässrigen Formaldehydlösung und sekundären Amin zeigen insbesondere im Rohrreaktor unter laminaren Strömungsbedingungen eine hohe Selektivität und eine gute Raum-Zeit-Ausbeute, sodass sich mit geringen Investitionskosten große Mengen kostengünstig produzieren lassen.

In einem zusätzlichen Verfahrensaspekt können je Mol Alkanal 0,01 bis 0,07 Äquivalente Carbonsäure zur Reaktion gebracht werden. Dieses Verhältnis Katalysator zu Alkanal hat sich zur Erreichung hoher Umsätze als besonders geeignet erwiesen. Anscheinend ist diese Menge ausreichend, um in Rohrreaktoren hohe Raum-Zeit-Ausbeuten bereitzustellen. Insbesondere kann durch dieses Verhältnis sowohl eine hinreichende Stabilisierung des laminaren Strömungsprofils sichergestellt werden als auch eine ausreichende Menge an Katalysator (aufgrund einer genügend hohen Beweglichkeit) innerhalb der Aldehydphase als katalytisches Zentrum für die Aldehydumsetzung fungieren. Je Mol Alkanal der Formel II im Einsatzprodukt können des Weiteren vorzugsweise 0,025 bis 0,05 Äquivalente sekundäres Amin und vorzugsweise auch 0,025 bis 0,05 Äquivalente Carbonsäure eingesetzt werden.

In einer bevorzugten Ausgestaltung des Verfahrens kann der Rest $R^1$ für einen aliphatischen C5-C13 Kohlenwasserstoffrest stehen. Das vorliegende Verfahren kann insbesondere dazu geeignet sein, diese mittelkettigen Aldehyde umzusetzen. Gerade mit den C5-C13 Aldehyden lassen sich sehr hohe Raum-Zeit-Ausbeuten realisieren, wobei der Anteil an nicht verwertbaren höhersiedenden Nebenprodukten äußerst gering ist. Ohne durch die Theorie gebunden zu sein, scheinen die Unterschiede in der Polarität der wässrigen Formaldehydphase und der Aldehyde dieser Gruppe derart geeignet zu sein, dass die Reaktion unter laminaren Strömungsbedingungen entlang der flüssig/flüssig Phasengrenze in ausreichendem Maße stattfindet. Insbesondere kann es vorteilhaft sein, nur ein oder mehrere Aldehyde gleicher C-Zahl in der Reaktion mit Formaldehyd einzusetzen. Trotz der relativ großen unpolaren C-Ketten ergeben sich hinreichende Reaktionsgeschwindigkeiten und eine gute Raum-Zeit-Ausbeute. Weiterhin kann es vorteilhaft sein, nur einen Aldehyd mit dieser Restdefinition in der Reaktionslösung vorliegen zu haben.

Innerhalb eines zusätzlichen Verfahrensaspekts kann das Molverhältnis Carbonsäure zu sekundärem Amin größer oder gleich 0,5 und kleiner oder gleich 2 betragen. Zum Erhalt einer insgesamt schnellen Reaktionskinetik und daraus folgend hoher Raum-Zeit-Ausbeuten hat sich das angegebene Verhältnis zwischen Carbonsäure und sekundärem Amin als besonders geeignet erwiesen.

In einer weiteren, bevorzugten Ausführungsform kann einer der eingesetzten Aldehyde n-Undecanal sein. Das erfindungsgemäße Verfahren eignet sich besonders zur Umsetzung von 1-Undecanal enthaltenden Gemischen, die bei der Hydroformulierung von Decen-1 erhalten werden. Je nach Hydroformylierungsbedingungen fallen wechselnde Verhältnisse an n-Undecanal zu 2-Methyldecanal an. In solchen Gemischen lässt sich nach dem erfindungsgemäßen Verfahren selektiv und in hoher Raum-Zeit-Ausbeute n-Undecanal in das 2-Methylenundecanal überführen, wobei 2-Methyldecanal aufgrund der geringeren Reaktivität unverändert bleibt und destillativ abgetrennt werden kann. Ebenfalls kann aus dem Hydroformulierungsgemisch zunächst n-Undecanal destillativ abgetrennt werden und anschließend nach dem erfindungsgemäßen Verfahren mit Formaldehyd umgesetzt werden. 2-Methylenundecanal kann durch Selektivhydrierung zum Beispiel am Palladium- oder Platinkontakt in 2-Methylundecanal derivatisiert werden, das ein wichtiger Rohstoff für die Reichstoffindustrie ist.

In vergleichbarer Weise kann z. B. aus dem Hydroformulierungsprodukt von Hexen-1 2-Methylenheptanal und anschließend 2-Methylheptanal gewonnen werden.

Weitere Einzelheiten, Merkmale und Vorteile des Gegenstandes der Erfindung ergeben sich aus den Unteransprüchen sowie aus der nachfolgenden Beschreibung der Figuren und der zugehörigen Beispiele. In den Figuren zeigt die:

Fig. 1 schematisch eine mögliche Ausgestaltung des erfindungsgemäßen Verfahrens unter Verwendung eines Rohrreaktors;

Fig. 2 schematisch eine weitere Ausführungsform des erfindungsgemäßen Verfahrens unter Verwendung eines einem Rohrreaktor vorgeschalteten Mischelements.

In der Figur 1 wird über Leitung (1) eine wässrige Formaldehydlösung, über Leitung (2) eine organische Lösung enthaltend ein sekundäres Amin und eine Carbonsäure als Katalysator für die Mannich-Kondensationsreaktion und über Leitung (3) frisches Alkanal, vermischt mit dem Kreislaufstrom, an dem Boden eines Rohrreaktors (4) eingeleitet. Das mit der wässrigen Formaldehydlösung zugeführte Wasser bildet die kontinuierliche Phase innerhalb des Rohrreaktors. Die über Leitung (2) herangeführte organische Lösung des Katalysators und das über Leitung (3) zugeführte Alkanal werden durch die am Boden des Rohrreaktors (4) installierten Vorrichtungen (5) in Flüssigkeitstropfen zerteilt und durchströmen als dispergierte organische Phase die kontinuierliche wässrige Phase auf Grund des Dichteunterschiedes in Richtung Reaktorkopf. Die Summe der zugeführten Massenströme ist so zu wählen, dass sich im Rohrreaktor ein laminarer Strömungszustand ausbildet. Zur Wärmeabfuhr können in dem Rohrreaktor Kühlschlangen oder Kühlfinger installiert sein, die jedoch nicht das laminare Strömungsverhalten der vereinigten Massenströme in Richtung Reaktorkopf stören (nicht in Figur 1 eingezeichnet). Am Reaktorkopf wird über Leitung (6) der flüssige Reaktoraustrag in einen Absetzbehälter (7) geleitet, in dem sich die leichtere organische Phase von der schweren wässrigen Phase trennt. Gasförmige Anteile werden über die Leitung (8) abgeführt. Die abgesetzte wässrige Phase, die noch Restmengen an Formaldehyd enthält, wird über Leitung (9) aus dem Prozess entfernt. Eine weitere Aufarbeitung der wässrigen Phase, z. B. durch Destillation, ist optional.

Die abgesetzte organische Phase, die das gewünschte 2-Methylenalkanal, nicht-umgesetztes Alkanal sowie ebenfalls nicht reagiertes 2-Methylalkanal enthält, wird über Leitung (10) aus dem Absetzbehälter abgeführt, von der eine Menge als Teilstrom über Leitung (11) abgezogen wird. Das abgezogene Rohprodukt kann anschließend aufgereinigt und für weitere Derivatisierungsreaktionen, wie zum Beispiel als Einsatzmaterial für die Selektivhydrierung verwendet werden. Es kann aber auch eine unmittelbare Derivatisierung mit daran anschließender Aufreinigung erfolgen.

Der nicht abgezogene Teilstrom kann über Leitung (12) als Kreislauf mit über Leitung (3) zugeführtem frischen Alkanal vereinigt und über Leitung (13) am Boden des Reaktors (4) gepumpt werden.

In der Figur 2 ist eine weitere Ausführungsform des erfindungsgemäßen Verfahrens unter Verwendung eines dem Rohrreaktor vorgeschalteten Mischelements dargestellt. Die über Leitung (1) herangeführte wässrige Formaldehydlösung, die über Leitung (2) herangeführte organische Lösung des Katalysators und das über Leitung (13) zugeführte Alkanal werden in dem statischen Mischer (14) dispergiert. Das Mehrphasengemisch tritt über Leitung (15) auf den Boden des Rohreaktors (4). Auf die schematisch angedeuteten Einbauten kann bei dieser Ausführungsform verzichtet werden, obwohl Vorrichtungen im Bodenbereich des Rohreaktors nicht ausgeschlossen sind, solange ein laminarer Strömungszustand des Rohreaktors sichergestellt ist.

Beispiele

In einen Rohreaktor mit einem Volumen von 0,191 Liter wird kontinuierlich über den Reaktorboden getrennt aber gleichzeitig jeweils eine wässrige Formalinlösung (30 Gew.-%), das Katalysatorgemisch (bestehend aus 95,0 g Di-n-Butylamin, 64,5 g n-Buttersäure, 50,1 g 2-Ethylhexanol) und der Aldehyd (Undecanal mit 67 % oder 91 % n-Undecanal-Anteil) eingespeist. Wird ohne statischen Mischer gearbeitet, kann man die Stoffströme getrennt aber gleichzeitig auf den Boden des Rohrreaktors leiten. Wird mit einem statischen Mischer gearbeitet (beispielsweise Sulzer Mischer vom Typ SMX DN4), so kann das Mischelement vor dem Reaktorboden außerhalb des Rohrreaktors angebracht werden. Darin können die wässrige Lösung sowie die organischen Lösungen miteinander vermischt und anschließend das flüssige Mehrphasensystem mit der dispergierten organischen Phase auf den Rohrreaktor gegeben werden. In beiden Ausführungsformen durchströmt die dispergierte organische Phase tropfenförmig die kontinuierliche wässrige Phase.

Am Reaktorkopf kann man das mehrphasige Reaktionsgemisch entnehmen und in einen Absetzbehälter führen. Aus den abgeschiedenen flüssigen Phasen wird ein organischer Kreislaufstrom wieder in den Rohrreaktor zurückgeführt.

Die nicht zurückgeführte wässrige Phase wird ausgeschleust, während die nicht zurückgeführte organische Phase gaschromatographisch auf ihren Wertproduktgehalt hin analysiert wurde.

Die Reaktionsbedingungen, die kontinuierliche Zufuhr der Einsatzstoffe sowie die Kreislaufströme wurden gemäß den Bedingungen der nachfolgenden Tabelle 1 eingestellt. In der Tabelle 1 ist ebenfalls die gaschromatographisch ermittelte Zusammensetzung des organischen Produkts, wasserfrei, angegeben in %, dargestellt. Die Versuche wurden bei einem Überdruck von 0,2 MPa betrieben.

Tabelle 1: Ergebnisse zur erfindungsgemäßen Umsetzung von Undecanal mit Formalin

| **Versuch** | **1** | **2** | **3** | **4** | **5** | **6** | **7** | **8** | **9** |
|---|---|---|---|---|---|---|---|---|---|
| Temperatur [°C] | 125 | 125 | 125 | 100 | 125 | 125 | 115 | 115 | 125 |
| Druck [bar] | 2,0 | 2,0 | 2,0 | 2,0 | 2,0 | 2,0 | 2,0 | 2,0 | 2,0 |
| V/Vh (Undecanal+Kreislauf) [1/h] | 14,2 | 14,3 | 14,3 | 14,3 | 13,8 | 13,3 | 13,8 | 13,7 | 13,7 |
| Formalin [g/h] | 47,0 | 47,0 | 47,0 | 46,7 | 58,0 | 28,0 | 58,0 | 84,0 | 84,0 |
| Undecanal [g/h] | 109,0 | 108,9 | 109,0 | 109,2 | 98,1 | 48,0 | 98,0 | 142,4 | 142,8 |
| Katalysatorlösung [g/h] | 2,9 | 4,5 | 5,9 | 5,8 | 7,0 | 4,5 | 7,9 | 10,1 | 10,4 |
| Kreislauf [g/h] | 2000 | 2000 | 2000 | 2000 | 2000 | 2000 | 2000 | 2000 | 2000 |
| Verh. n-C11-al zu Formalin | 1-1,09 | 1-1,09 | 1-1,09 | 1-1,09 | 1-1,09 | 1-1,09 | 1-1,09 | 1-1,09 | 1-1,09 |
| Verh. n-C11-al zu Bu2NH | 1-0,025 | 1-0,04 | 1-0,05 | 1-0,05 | 1-0,05 | 1-0,05 | 1-0,05 | 1-0,05 | 1-0,05 |
| Verh. Bu2NH zu n-C4-Säure | 1-1 | 1-1 | 1-1 | 1-1 | 1-1 | 1-1 | 1-1 | 1-1 | 1-1 |
| %-Anteil n-Undecanal im Undecanal | 67 | 67 | 67 | 67 | 91 | 91 | 91 | 91 | 91 |
| **Analyse des Produktstromes [%]** | | | | | | | | | |
| Vorlauf | 0,36 | 0,05 | 0,04 | 0,05 | 0,05 | 0,05 | 0,03 | 0,06 | 0,11 |
| n-Buttersäure | 0,40 | 0,60 | 0,81 | 0,87 | 0,81 | 0,84 | 0,83 | 0,94 | 0,91 |
| Zwischenlauf 1 | 0,17 | 0,16 | 0,16 | | | | | | |
| n-Decan max. | 0,08 | 0,08 | 0,07 | 0,05 | | < 0,01 | 0,02 | | |
| Decen-Bereich | 0,29 | 0,32 | 0,27 | 0,43 | 0,26 | 0,22 | 0,32 | 0,22 | 0,37 |
| 2-EH-ol | 0,77 | 1,08 | 1,40 | 1,40 | 1,86 | 1,77 | 1,66 | 1,70 | 1,60 |
| Zwischenlauf 2 | 0,24 | 0,24 | 0,23 | 0,19 | 0,45 | 0,16 | 0,39 | 0,27 | 0,30 |
| 2-EH-Säure | 0,03 | 0,03 | 0,03 | 0,02 | | | | | |
| 2-Butylheptanal | 0,05 | 0,05 | 0,04 | 0,04 | 0,01 | < 0,01 | 0,01 | | 0,01 |
| 2-Propyloctanal | 0,08 | 0,07 | 0,07 | 0,07 | 0,01 | 0,01 | 0,01 | 0,01 | 0,01 |
| 2-Ethylnonanal | 0,82 | 0,78 | 0,72 | 0,83 | 0,21 | 0,20 | 0,19 | 0,21 | 0,19 |
| 2-Methyldecanal | 26,48 | 26,10 | 25,11 | 26,93 | 4,34 | 4,87 | 4,35 | 4,55 | 4,14 |
| Zwischenlauf 3 | 0,26 | 0,25 | 0,33 | 0,37 | 0,15 | 0,18 | 0,20 | 0,16 | 0,16 |

(fortgesetzt)

| Analyse des Produktstromes [%] | | | | | | | | | |
|---|---|---|---|---|---|---|---|---|---|
| n-Undecanal | 10,72 | 8,98 | 6,10 | 10,60 | 1,53 | 2,73 | 0,82 | 2,49 | 1,36 |
| Zwischenlauf 4 | 3,82 | 3,72 | 4,67 | 0,05 | 0,07 | 0,07 | 0,06 | 0,11 | 0,10 |
| 2-Methyldecanol | 0,12 | 0,11 | 0,11 | 0,12 | 0,04 | 0,04 | 0,05 | 0,02 | 0,03 |
| 2-Methylenundecanal | 25,06 | 28,28 | 36,96 | 27,54 | 68,20 | 66,05 | 71,22 | 64,45 | 69,31 |
| 2-Hydroxymethylundecanal | 25,45 | 24,70 | 18,44 | 24,36 | 5,43 | 2,59 | 5,14 | 12,39 | 10,73 |
| Nachlauf | 4,80 | 4,40 | 4,44 | 6,08 | 16,58 | 20,22 | 14,70 | 12,42 | 10,67 |
| Summe | 100 | 100 | 100 | 100 | 100 | 100 | 100 | 100 | 100 |
| Umsatz n-Undecanal | 84% | 87% | 91% | 84% | 98% | 97% | 99% | 97% | 99% |
| Selektivität* | 90% | 91% | 91% | 92% | 82% | 77% | 84% | 86% | 89% |
| Ausbeute* | 75% | 79% | 83% | 77% | 81% | 75% | 84% | 84% | 88% |
| * = gebildete Menge 2-Methylenundecanal+2-Hydroxymethylundecanal in % bez. auf die eingesetzte Menge an n-Undecanal | | | | | | | | | |

Aus der Tabelle 1 wird deutlich, dass im Rahmen eines kontinuierlichen Herstellungsprozesses, innerhalb eines Rohrreaktors in laminarer Fahrweise durch Säure-Base-Katalyse hohe Ausbeuten erhältlich sind. Die Ausbeuten liegen sämtlich oberhalb von 75%, wobei generell hohe Umsätze größer als 84% realisiert werden. Diese ergeben in Summe hohe Raum-Zeit-Ausbeuten, welche in dieser Größenordnung weder durch eine Batch- noch durch eine kontinuierliche Fahrweise in Rührkesseln/-Kaskaden erhältlich sind.

Durch Variation des gesamten Massenstromes der Reaktanden kann die Reynolds-Zahl gezielt so eingestellt werden, dass sich ein laminarer Strömungszustand im Rohrreaktor einstellt. Für die Versuche mit der höchsten Reaktorbelastung (Versuche 8 und 9 in Tabelle 1) liegt die Reynolds-Zahl bei 176 (vgl. Tabelle 2) und somit im laminaren Strömungsbereich (kleiner als 2320). In den Beispielen 1-7 wurde mit einem geringeren Einsatzmassenstrom gearbeitet. Demzufolge ergibt sich aus Gleichung (1) eine kleinere Reynolds-Zahl. In den aufgeführten Beispielen liegen somit stabile laminare Strömungsbedingungen vor.

Tabelle 2: Hydraulische Kennzahlen, exemplarisch für Beispiel Versuch 8:

| | | |
|---|---|---|
| Dichte $\rho$ | [$kg/m^3$] | 990 |
| Dynamische Viskosität $\eta$ | [Pa s] | 0,0005 |
| Hydraulischer Innendurchmesser d | [m] | 0,009 |
| Massenstrom der Reaktanden | [kg/h] | 2,24 |
| Reynolds-Zahl | | 176 |

Das aus den Versuchen erhaltene Reaktionsprodukt kann in nachfolgenden Reinigungsschritten aufgetrennt und anschließend am Palladium- oder Platinkatalysator partiell hydriert werden. 2-Methyldecanal liegt im hydrierten Reaktionsgemisch unverändert vor und das gewünschte 2-Methylundecanal kann fraktioniert abdestilliert werden. 2-Methylundecanal ist ein wertvolles Produkt für die Riechstoffindustrie.

## Patentansprüche

1. Verfahren zur Herstellung von 2-Methylenalkanalen der allgemeinen Formel I

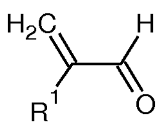

Formel I,

worin $R^1$ für einen aliphatischen Rest steht, durch Umsetzung von Alkanalen nach der allgemeinen Formel II

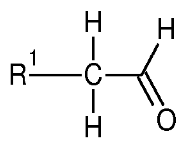

Formel II,

mit einer wässrigen Formaldehydlösung in Gegenwart mindestens eines sekundären Amins und mindestens einer Carbonsäure, **dadurch gekennzeichnet, dass** die Edukte in einem flüssig/flüssig Zweiphasensystem zur Reaktion gebracht werden, wobei die Reaktion kontinuierlich innerhalb eines Rohrreaktors unter laminaren Strömungsbedingungen mit einer Reynolds-Zahl von größer oder gleich 10 und kleiner oder gleich 2320 durchgeführt wird.

2. Verfahren nach Anspruch 1, wobei die Reynolds-Zahl innerhalb des Rohrreaktors konstant gehalten wird.

3. Verfahren nach Anspruch 1 oder 2, wobei das flüssig/flüssig Zweiphasensystem durch getrenntes Einspeisen im Gleichstrom mindestens einer organischen und mindestens einer wässrigen Phase in den Rohrreaktor ausgebildet wird.

4. Verfahren nach einem der vorhergehenden Ansprüche, wobei die Carbonsäure zusammen mit dem sekundären

Amin in einem organischen Lösungsmittel gelöst in den Reaktor eingespeist wird.

5. Verfahren nach Anspruch 4, wobei das Lösungsmittel ausgesucht ist aus der Gruppe der Monoalkohole mit 6 bis 12 Kohlenstoffatomen oder Mischungen daraus.

6. Verfahren nach einem der vorhergehenden Ansprüche, wobei die Reaktion bei einer Temperatur von größer oder gleich 70 °C und kleiner oder gleich 150°C durchgeführt wird.

7. Verfahren nach einem der vorhergehenden Ansprüche, wobei in dem Zweiphasensystem keine zusätzlichen oberflächenaktiven Substanzen vorliegen.

8. Verfahren nach einem der vorhergehenden Ansprüche, wobei der Rohrreaktor mit einer Reynolds-Zahl von größer oder gleich 50 und kleiner oder gleich 500 betrieben wird.

9. Verfahren nach einem der vorhergehenden Ansprüche, wobei die Reaktorbelastung V/Vh bezogen auf den Gesamtmassenstrom an Reaktanden größer oder gleich 3,0 $h^{-1}$ und kleiner oder gleich 42,0 $h^{-1}$ beträgt.

10. Verfahren nach einem der vorhergehenden Ansprüche, wobei die Carbonsäure ausgesucht ist aus der Gruppe der aliphatischen oder aromatischen C2-C12 Monocarbonsäuren.

11. Verfahren nach einem der vorhergehenden Ansprüche, wobei das molare Verhältnis aus Alkanal, Formaldehyd im Formalin und sekundärem Amin im Bereich von 1 zu 1 zu 0,01 bis 1 zu 1,2 zu 0,07 liegt.

12. Verfahren nach einem der vorhergehenden Ansprüche, wobei der Rest $R^1$ für einen aliphatischen C5-C13 Kohlenwasserstoffrest steht.

13. Verfahren nach einem der vorhergehenden Ansprüche, wobei mindestens zwei unterschiedliche Alkanale mit gleicher C-Zahl und C größer oder gleich 2 zur Reaktion gebracht werden, wobei mindestens eines der Alkanale eine Struktur nach Formel II aufweist.

14. Verfahren nach einem der vorhergehenden Ansprüche, wobei das Molverhältnis Carbonsäure zu sekundärem Amin größer oder gleich 0,5 und kleiner oder gleich 2 beträgt.

15. Verfahren nach einem der vorhergehenden Ansprüche, wobei einer der eingesetzten Aldehyde n-Undecanal ist.

## Claims

1. Method for the production of 2-methylene aldehydes of General Formula I

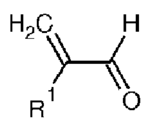

Formula I,

wherein $R^1$ denotes an aliphatic radical, by reacting aldehydes according to General Formula II

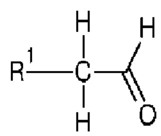

Formula II,

with an aqueous formaldehyde solution in the presence of at least one secondary amine and at least one carboxylic acid, **characterized in that** the reactants are reacted in a liquid-liquid two-phase system, wherein the reaction is carried out continuously inside a tubular reactor under laminar flow conditions with a Reynolds number greater than or equal to 10 and less than or equal to 2320.

2. Method according to Claim 1, wherein the Reynolds number inside the tubular reactor is kept constant.

3. Method according to Claim 1 or 2, wherein the liquid-liquid two-phase system is formed by separate feeding in co-current flow of at least one organic and at least one aqueous phase into the tubular reactor.

4. Method according to one of the preceding claims, wherein the carboxylic acid dissolved together with the secondary amine in an organic solvent is fed into the reactor.

5. Method according to Claim 4, wherein the solvent is selected from the group of the monoalcohols with 6 to 12 carbon atoms or mixtures thereof.

6. Method according to one of the preceding claims, wherein the reaction is carried out at a temperature greater than or equal to 70°C and less than or equal to 150°C.

7. Method according to one of the preceding claims, wherein no additional surface-active substances are present in the two-phase system.

8. Method according to one of the preceding claims, wherein the tubular reactor is operated with a Reynolds number greater than or equal to 50 and less than or equal to 500.

9. Method according to one of the preceding claims, wherein the reactor load V/Vh based on the total mass flow of the reactants is greater than or equal to 3.0 $h^{-1}$ and less than or equal to 42.0 $h^{-1}$.

10. Method according to one of the preceding claims, wherein the carboxylic acid is selected from the group of the aliphatic or aromatic C2-C12 monocarboxylic acids.

11. Method according to one of the preceding claims, wherein the molar ratio of aldehyde, formaldehyde in the formalin and secondary amine is in the range of (1 to 1 to 0.01) to (1 to 1.2 to 0.07).

12. Method according to one of the preceding claims, wherein the radical $R^1$ denotes an aliphatic C5-C13 hydrocarbon radical.

13. Method according to one of the preceding claims, wherein at least two different aldehydes with the same C number and C greater than or equal to 2 are reacted, wherein at least one of the aldehydes has a structure according to Formula II.

14. Method according to one of the preceding claims, wherein the molar ratio of carboxylic acid to secondary amine is greater than or equal to 0.5 and less than or equal to 2.

15. Method according to one of the preceding claims, wherein one of the aldehydes used is n-undecanal.

**Revendications**

1. Procédé de fabrication de 2-méthylène-alcanals de formule générale I

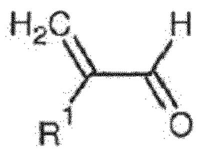

Formule I,

dans laquelle $R^1$ représente un radical aliphatique, par mise en réaction d'alcanals de formule générale II

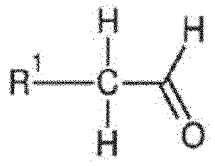

Formule II

avec une solution aqueuse de formaldéhyde en présence d'au moins une amine secondaire et d'au moins un acide carboxylique, **caractérisé en ce que** les réactifs sont mis en réaction dans un système biphasé liquide/liquide, la réaction étant réalisée en continu dans un réacteur tubulaire dans des conditions d'écoulement laminaire avec un nombre de Reynolds supérieur ou égal à 10 et inférieur ou égal à 2 320.

2. Procédé selon la revendication 1, dans lequel le nombre de Reynolds dans le réacteur tubulaire est maintenu constant.

3. Procédé selon la revendication 1 ou 2, dans lequel le système biphasé liquide/liquide est formé par introduction séparée à co-courant d'au moins une phase organique et d'au moins une phase aqueuse dans le réacteur tubulaire.

4. Procédé selon l'une quelconque des revendications précédentes, dans lequel l'acide carboxylique est introduit dans le réacteur conjointement avec l'amine secondaire, dissous dans un solvant organique.

5. Procédé selon la revendication 4, dans lequel le solvant est choisi dans le groupe constitué par les monoalcools de 6 à 12 atomes de carbone ou les mélanges de ceux-ci.

6. Procédé selon l'une quelconque des revendications précédentes, dans lequel la réaction est réalisée à une température supérieure ou égale à 70 °C et inférieure ou égale à 150 °C.

7. Procédé selon l'une quelconque des revendications précédentes, dans lequel aucune substance tensioactive supplémentaire n'est présente dans le système biphasé.

8. Procédé selon l'une quelconque des revendications précédentes, dans lequel le réacteur tubulaire est exploité avec un nombre de Reynolds supérieur ou égal à 50 et inférieur ou égal à 500.

9. Procédé selon l'une quelconque des revendications précédentes, dans lequel le chargement du réacteur V/Vh par rapport au débit massique total des réactifs est supérieur ou égal à 3,0 $h^{-1}$ et inférieur ou égal à 42,0 $h^{-1}$.

10. Procédé selon l'une quelconque des revendications précédentes, dans lequel l'acide carboxylique est choisi dans le groupe constitué par les acides monocarboxyliques en C2-C12 aliphatiques ou aromatiques.

11. Procédé selon l'une quelconque des revendications précédentes, dans lequel le rapport molaire entre l'alcanal, le formaldéhyde dans de la formaline et l'amine secondaire se situe dans la plage allant de 1 sur 1 sur 0,01 à 1 sur 1,2 sur 0,07.

12. Procédé selon l'une quelconque des revendications précédentes, dans lequel le radical $R^1$ représente un radical hydrocarboné en C5-C13 aliphatique.

13. Procédé selon l'une quelconque des revendications précédentes, dans lequel au moins deux alcanals différents contenant le même nombre de C et ayant un nombre de C supérieur ou égal à 2 sont mis en réaction, au moins un des alcanals présentant une structure selon la formule II.

14. Procédé selon l'une quelconque des revendications précédentes, dans lequel le rapport molaire entre l'acide carboxylique et l'amine secondaire est supérieur ou égal à 0,5 et inférieur ou égal à 2.

15. Procédé selon l'une quelconque des revendications précédentes, dans lequel un des aldéhydes utilisés est le n-undécanal.

**Figur 1:**

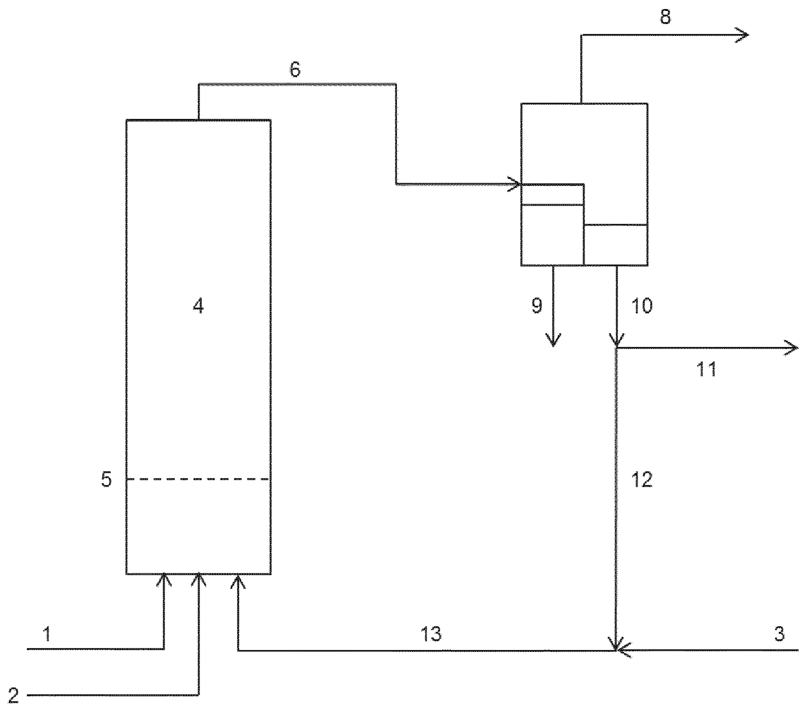


**Figur 2:**

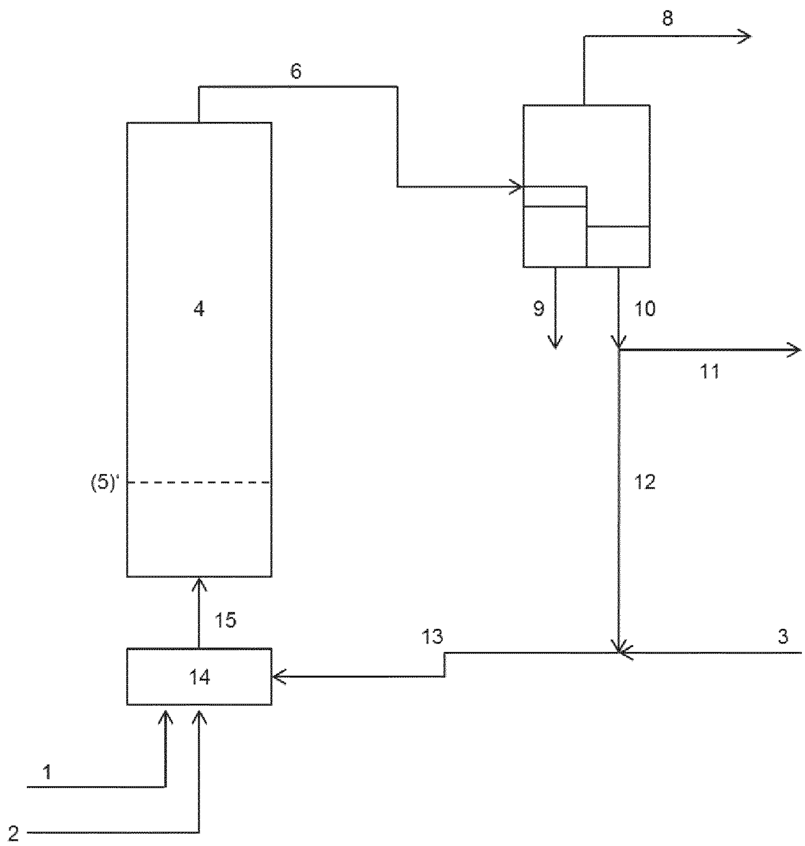

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- DE 2855506 A1
- WO 199320034 A1
- DE 3744212 A1
- DE 19957522 A1
- EP 2998284 A1
- WO 2014170223 A1
- EP 2883859 A1
- DE 19957522
- US 4263460 A
- WO 2013079614 A1
- CN 105693491
- WO 2010105892 A1


### In der Beschreibung aufgeführte Nicht-Patentliteratur


- **HOUBEN WEYL.** Methoden der Organischen Chemie. Georg Thieme Verlag, 1954, vol. VII, 93-94
- Ullmans's Encyclopedia of Industrial Chemistry. VCH Verlagsgesellschaft mbH, 1985, vol. A1, 326-331
- ULLMANS'S ENCYCLOPEDIA OF INDUSTRIAL CHEMISTRY. VCH Verlagsgesellschaft mbH, 1991, vol. A18, 321-327
- **MATTHIAS KRAUME.** Transportvorgänge in der Verfahrenstechnik: Grundlagen und apparative Umsetzungen. Springer, 2012
- Aarau und Frankfurt am Main. **HEINZ BAUER.** Grundlagen der Einphasen- und Mehrphasenströmungen. Verlag Sauerländer, 1971